# EUROPEAN PATENT SPECIFICATION

(11) **EP 2 919 721 B1**
(45) Date of publication and mention of the grant of the patent: **02.12.2020**
(21) Application number: 13818364.5
(22) Date of filing: 16.10.2013
(51) Int. Cl.: A61F 5/01

(54) **HINGED ANKLE FOOT ORTHOSIS**
SCHWENKBARE FUSSGELENKORTHESE
ORTHÈSE CHEVILLE-PIED À CHARNIÈRE

(30) Priority: 18.10.2012 IT BA20120060
(43) Date of publication of application: 23.09.2015
(73) Proprietor: Vittore, Francesco, 70124 Bari (IT); O.T.P. Ortopuglia S.r.l., 70124 Bari (IT)
(72) Inventor: VITTORE, Francesco, I-701424 Bari (IT)
(74) Representative: Russo, Dimitri
(86) International application number: PCT/IB2013/002303
(87) International publication number: WO 2014/060824

(56) References cited:
- US-A- 3 779 654
- US-A- 5 086 760
- US-A- 5 094 232
- US-A- 5 865 778

## Description

The object of the present invention is a leg-foot orthosis, hinged at the ankle to be used in the functional recovery phase after a traumatic event or in case of injury to the foot bones or to the capsular ligament apparatus of the ankle and of the rearfoot.

As it is known, the articulation of the ankle, also called tibiotarsal articulation, is an angular ginglymoid articulation that joins the tibia and the fibula, which form a concave surface called "tibiofibular mortise," with the trochlea of the astragalus. The articular surfaces are made up of the inferior articular facet of the tibia and the articular facets of the medial and lateral malleolus. The astragalus participates with the trochlea and with the lateral and medial malleolar facets. Jointing means of the tibiotarsal articulation are represented by the articular capsule, which is arranged on the contours of the tibiofibular mortise and on the margins of the articular cartilage of the astragalus, and by reinforcing ligaments, which are the medial or deltoid ligament and the anterolateral, lateral and rear ligaments. The tibiotarsal articulation, due to its structure, allows the foot to make movements of dorsal and plantar flexion; in particular, the flexion angle of the foot, assessed in a plane parallel to the median sagital plane, has a width of about 70°.

In the course of a dorsal or plantar flexion movement of the ankle, the articular surface of the tibia and the malleoli (tibial and peroneal) moves with a roto-translatory type of motion.

Such a roto-translatory motion can be highlighted and confirmed via identification of the instantaneous centers of rotation, i.e. those points defined by the Chasles Theorem, which affirms that in the course of a rotary movement around a fixed axis or a roto-translatory motion the straight line conducted through each point that is integral to the body in motion and perpendicular to the velocity vector contains a point called "center of instantaneous rotation", which is characterized by having zero velocity.

In case of a tibiotarsal articulation under physiological conditions, the centers of instantaneous rotation are located on a plane parallel to the median sagital plane, and in particular in an area belonging to the lateral surface of the astragalar body, and confirms that the motion occuring is of the roto-translatory type. If the centers of instantaneous rotation were to coincide at a single point, there would be a purely rotary motion around an axis normal to the plane and passing through said point, as well as the unique center of instantaneous rotation. Therefore, a pure rotary motion around a unique point does not represent a natural physiological movement made by the ankle under physiological conditions, which, by contrast, will present an area in which to locate the centers of instantaneous rotation.

Following traumas of the ankle, injury to the foot bones or to the capsular ligament apparatus of the ankle and of the rearfoot, it is typical to immobilize the articulation by use of the so-called orthosis, which are also necessary for the treatment and rehabilitation of the post-traumatic pathologies.

The orthosis normally used fall into three categories: rigid, semi-rigid or elastic and articulated at the ankle. However, the rigid-type orthosis present a problem, or rather the impossibility to make articular motions of the ankle and the foot to which it is applied.

By contrast, the orthosis of the semi-rigid or elastic type do not guarantee any protection with regard to the stress to which the capsular-ligament apparatus is subjected in the course of supination and pronation movements.

Finally, the orthosis hinged at the ankle characterized by the possibility of blocking according to a desired flexion - extension angulation and by the good protection in pronosupination, also present a problem. In particular, the elements of the aforementioned orthosis are connected by a mono-axial hinge, which allows only unnatural movements of the rotary type and does not permit the tibia to make the physiological sliding movement on the astragalar surface, which is described by a roto-translatory motion that is characterized by a series of instantaneous centers of rotation. Furthermore, the use of orthosis hinged at the ankle generates a series of problems for the ligament apparatus to be healed, whose articular surface and whose cartilage coat will be subject to compressive stresses that could result in their further injury.

Some examples of previous hinged ankle foot orthosis are disclosed in the following Patents. More in details, the US patent N° US 5 094 232 A (Harris David P [US] et al) 10 March 1992, discloses an ankle brace for limiting flexing and pivoting movement of the ankle joint, while the US patent N° US 5 865 778 A (Johnson James F [US]) 2 February 1999, discloses a pivoting ankle brace for conventional footwear. The US patent N° US 5 086 760 A (Neumann Holm W [US] et al) 11 February 1992, describes an articulated, orthotic brace for providing support to an anatomical joint.

All the devices disclosed in the above patents comprise a single mono-axial mechanical joint, which prevents the tibia to make the physiological sliding movement on the astragalar surface, which is described by a roto-translatory motion, characterized by a series of instantaneous centers of rotation.

Another previous hinged ankle foot orthosis is disclosed in the US patent N° US 377 965 4 (Robert V. Horne [US] et al) 18 December 1973. This orthosis comprises an artificial joint for simulating motion of a natural slide and hinge joint of the body, such as those at the knee, elbow or ankle. The artificial joint includes two rigid overlapping plates connected by a pivot and slide arrangement comprising two pivots, each pivot sliding in one of two opposite curved grooves facing each other.

The orthosis that is the object of the present invention therefore offers a series of solutions to address the drawbacks of the orthoses of the known type.

The principle objective of the present invention is to provide a leg-foot orthosis hinged at the ankle that is equipped with a hinge mechanism that joins its main elements and allows the foot to make natural flexion - extension movements according to a roto-translatory motion.

A further objective of the present invention is to provide a leg-foot orthosis, hinged at the ankle, which does not allow the foot to make pronosupination, adduction or abduction movements if they were to be considered damaging to the functional recovery of the articulation.

A final objective of the present invention is to provide a leg-foot orthosis, hinged at the ankle, that allows the foot to make natural flexion - extension movements limited to a given degree value.

These and other objectives are achieved by the leg-foot orthosis, hinged at the ankle, to be used in the functional recovery phase after a traumatic event or in case of injury to the foot bones or the capsular ligament apparatus of the ankle and of the rearfoot, which is defined in independent claim 1. Preferred embodiments are defined in the dependent claims.

The embodiment described below is preferred embodiment that does not limit further developments within the scope of the invention itself with the aid of the five drawings that illustrate the following figures:
- Fig. 1: an exploded view of the components of the orthosis hinged at the ankle;
- Fig. 2: an axonometric view of the orthosis in the phase of application to the ankle;
- Fig. 3: an axonometric view of the orthosis applied to the ankle;
- Fig. 4: a plan view of the first stirrup of the orthosis;
- Fig. 5: a plan view of the orthosis connected to the leg holder;
- Fig. 6: a plan view of the stirrup connected to the heel shell;
- Fig. 7: an exploded plan view of the hinge mechanism in a different configuration.

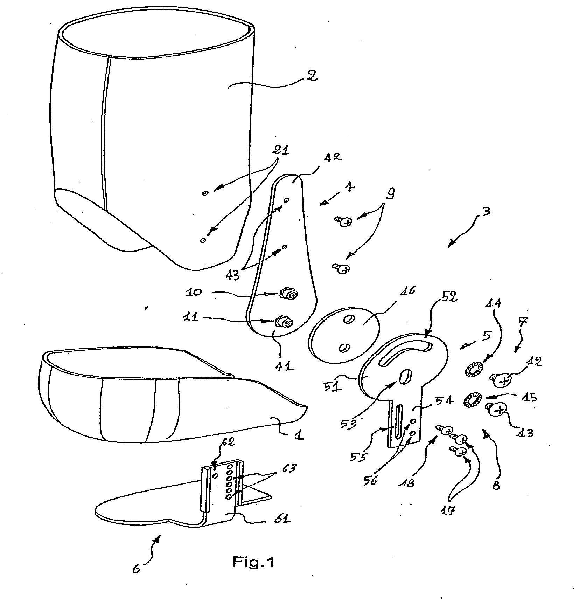

With reference to figures 1, 2 and 3, the orthosis, hinged at the ankle, comprises a heel shell 1 made of a polymer material and having a shape suited to be in contact with the heel T of the patient foot, and a rigid leg holder 2 made of a polymer material that is worn and firmly locked at the lower part of the leg G.

The heel shell 1 and the leg holder 2 are joined to each other via a hinge mechanism 3 comprising a first stirrup 4, a second stirrup 5 and a final press-braked L-stirrup 6, joined to each other via a series of threaded connections and mechanical constraints.

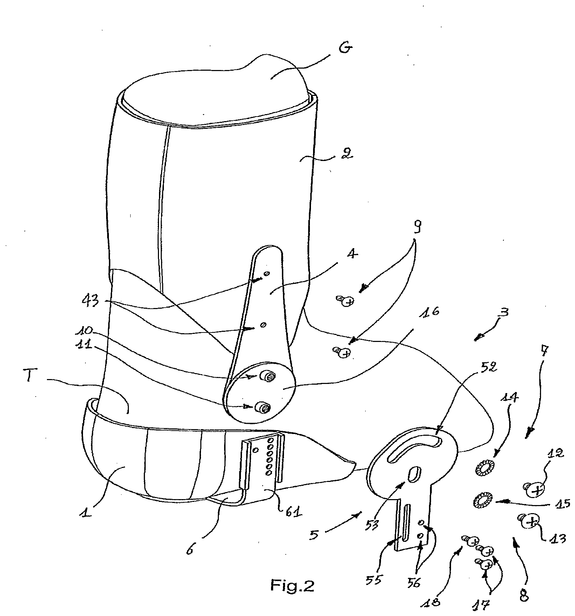

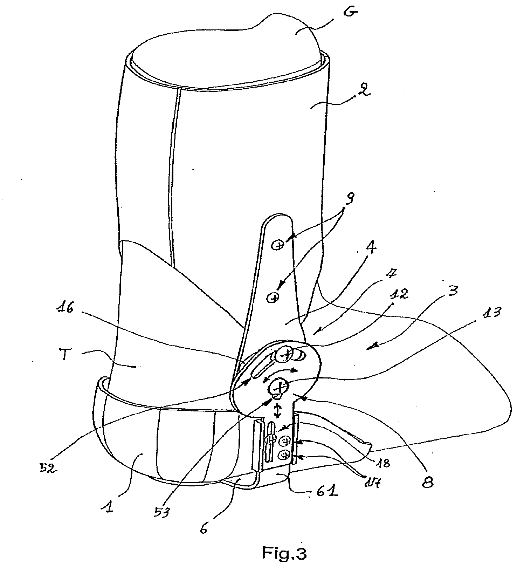

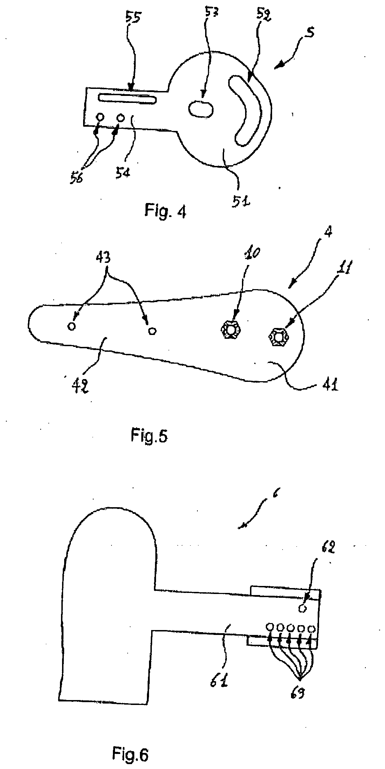

In particular, stirrup 4, as shown in detail in Fig. 5, at circular end 41, has a pair of threaded cylindrical rivets 10 and 11 of a thickness suitable for guaranteeing a sufficient hold, and at end 42 has a pair of holes 43 by means of which it is possible to join stirrup 4 to leg holder 2 by means of a pair of screws 9 (Figs. 2 and 3).

At its circular end 51, stirrup 5 has a through-slot 52 shaped as consecutive circumferential arcs and, perpendicular to it, a slotted hole 53. At end 54 of stirrup 5, of the characteristic rectangular shape, are another slot 55 and, parallel to it, a pair of holes 56.

As shown in Figures 2 and 3, stirrup 5 is hinged to stirrup 4 by means of a first pin 7, comprising a screw 12 screwed into the upper threaded cylindrical rivet 10 of stirrup 4, and joined so as to slide within slot 52 of stirrup 5, and a second pin 8, comprising a screw 13 that is screwed into the lower threaded cylindrical rivet 11 and joined so as to slide within the slotted hole 53 of stirrup 5. Interposed between screws 12 and 13 and stirrup 5 are washers with external serration 14 and 15 necessary for locking the hinge mechanism 3 in a specific desired angulation. In addition, to facilitate the reciprocal sliding of stirrups 4 and 5, a disk 16 made of Teflon or other material suitable for reducing abrasion between the two elements is interposed between the two elements.

Finally, as shown in Figure 3, another press-braked L-stirrup 6, which is already engaged to the heel shell 1 by gluing or other process, is connected to stirrup 5 by means of a pair of screws 17 and a screw 18 passing through holes 56 and slot 55 of stirrup 5 and screwed within the threaded holes 62 and 63 located on tab 61 of L-stirrup 6.

Threaded holes 62 and 63 of the L-stirrup 6 are necessary for fixing stirrup 5 in the desired position. Fixation in the desired position is made possible by means of holes 56 and slot 55, which allow locking in place stirrup 5 at the tibio-astragalar articular rima.

As shown in Fig. 3, following the assembly of all of the elements of the hinge mechanism 3, heel shell 1 and leg holder 2 will be joined to each other via hinge mechanism 3 comprising stirrups 4, 5 and 6, and will be able to make reciprocal and simultaneous roto-translatory movements in space, describing the physiological movement of the tibiotarsal articulation.

In particular, slot 52 allows the upper pin 7 to be guided when making the flexion - extension movement, and, simultaneously, lower pin 8 as a consequence of the motion will be positioned in axis having the instantaneous centers of rotation within slotted hole 53.

The movement of the hinge mechanism 3, instant by instant, identifies two instantaneous centers of rotation corresponding to the perpendicular lines at the speed vector of pins 7 and 8.

In a different configuration (not depicted in the figures), the first stirrup 4 and the leg holder 2 may consist of a leg holder 2 on whose lateral surface are present suitable elements that enable leg holder 2 to be joined to heel shell 1 via the hinge mechanism described above. Similarly, the second stirrup 5, the L-stirrup 6 and the heel shell 1 may comprise a heel shell 1 on whose lateral surface are present suitable elements that enable heel shell 1 to be joined to leg holder 2 via the hinge mechanism described above.

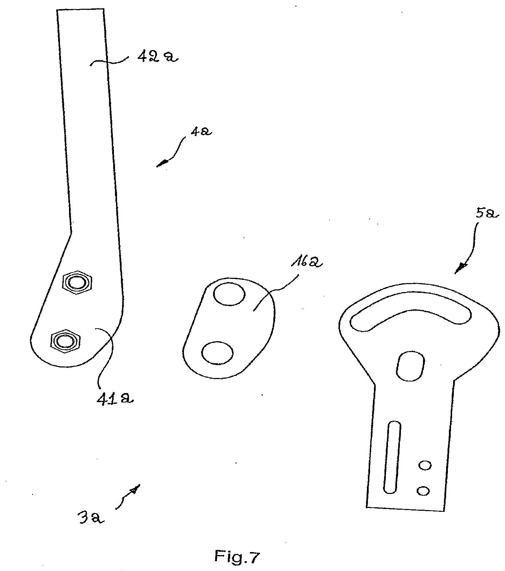

With reference to Fig. 7, hinge mechanism 3a can be made in a different configuration; in particular, it will comprise a stirrup 4a connected to leg holder 2 on whose elliptical end 41a, which is inclined at about 20° with respect to end 42a, a pair of threaded cylindrical rivets will be present as well.

Hinge mechanism 3a further comprises a stirrup 5a on which are present the same slots and holes of stirrup 5, the spacer disk of elliptical shape 16a and L-bracket 6 (not depicted in Fig. 7).

## Claims

1. *Leg-foot orthosis, hinged at the ankle, to be employed in the functional recovery phase after a traumatic event or in case of injury to the foot bones or to the capsular ligament apparatus of the ankle and of the rearfoot,* comprising a heel shell (1), a leg holder (2) and a hinge mechanism (3) engaged with said heel shell (1) and said leg holder (2), the hinge mechanism (3) comprising a first stirrup (4) and a second stirrup (5), which are mechanically articulated by means of a first pin (7) and a second pin (8) respectively engaged in apertures (52, 53) provided in the stirrups (4, 5),
**characterized in that** the aperture (52) in which the first pin (7) is engaged is a first slot (52) shaped as consecutive circumferential arcs, and the aperture (53) in which the second pin (8) is engaged is a slotted hole (53), the slotted hole running transverse to said first slot (52), in such a way that said hinge mechanism (3) is adapted to enable said heel shell (1) and said leg holder (2) to make respective simultaneous roto-translatory movements in space.

2. Leg-foot orthosis, hinged at the ankle according to claim 1, wherein the first stirrup (4) is connected via screws (9) or suitable fastening devices to the leg holder (2),
wherein the second stirrup (5) comprises, on its surface, the single arcuate slot (52), the slotted hole (53), a pair of holes (56) and a slot (55),
wherein the leg-foot orthosis further comprises an L-stirrup (6), being engaged with the heel shell (1) and comprising a tab (61) on which are present a series of holes (63) and one hole (62) parallel to them, and
wherein the pair of pins (7, 8) consist of a pair of threaded cylindrical rivets (10, 11) provided on the first stirrup (4), a pair of screws (12, 13) and a pair of washers with external serration (14, 15).

3. Leg-foot orthosis, hinged at the ankle, according to claim 1, further comprising, between the stirrups (4, 5) of the hinge mechanism (3), a spacer disk (16) made of teflon or other material adapted to induce a friction reduction between the surfaces of the stirrups (4, 5).

4. Leg-foot orthosis, hinged at the ankle, according to claim 2, wherein the stirrup (5) is connected to the L-stirrup (6), and thus to heel shell (1), via a pair of screws (17) engaged through the pair of holes (56) of the stirrup (5), and a screw (18) joined at slot (55) of stirrup (5), said screws (17, 18) being suitably screwed within the holes (62, 63) of the L-stirrup (6).

5. Leg-foot orthosis, hinged at the ankle, according to one or more of the preceding claims, wherein the first stirrup (4) and the leg holder (2) consist of a leg holder (2) on whose lateral surface are present suitable elements that enable leg holder (2) to be joined to heel shell (1) via the hinge mechanism.

6. Leg-foot orthosis, hinged at the ankle, according to one or more of the preceding claims, wherein the second stirrup (5), the L-stirrup (6) and the heel shell (1) comprise a heel shell (1) on whose lateral surface are present suitable elements that enable said heel shell (1) to be joined to leg holder (2) via the hinge mechanism.

7. Leg-foot orthosis, hinged at the ankle, according to one or more of the preceding claims, wherein the first stirrup (4) is a stirrup (4a) having an elliptical end (41a) which is inclined at approximately 20° with respect to the other end (42a), the second stirrup (5a) comprising suitable fastening elements, and further comprising an elliptical spacer disk (16a).

## Patentansprüche

1. *Bein-Fuß-Orthese, die am Knöchel angelenkt ist, zum Einsatz in der funktionellen Erholungsphase nach einem traumatischen Ereignis oder im Falle einer Verletzung der Fußknochen oder des Kapselbandapparats des Knöchels und des Rückfußes,* umfassend eine Fersenschale (1), einen Beinhalter (2) und einen Scharniermechanismus (3), der mit der Fersenschale (1) und dem Beinhalter (2) in Eingriff steht, wobei der Scharniermechanismus (3) einen ersten Bügel (4) und einen zweiten Bügel (5) umfasst, die mittels eines ersten Stifts (7) und eines zweiten Stifts (8) mechanisch angelenkt sind, die jeweils in Öffnungen (52, 53) im Eingriff stehen, die in den Bügeln (4, 5) bereitgestellt sind, **dadurch gekennzeichnet, dass** die Öffnung (52), in der der erste Stift (7) im Eingriff steht, ein erster Schlitz (52) ist, der als aufeinanderfolgende Umfangsbögen geformt ist, und die Öffnung (53), in der der zweite Stift (8) im Eingriff steht, ein Schlitzloch (53) ist, wobei das Schlitzloch derart quer zum ersten Schlitz (52) verläuft, dass der Scharniermechanismus (3) dafür konzipiert ist, der Fersenschale (1) und dem Beinhalter (2) ein Durchführen von jeweiligen gleichzeitigen Drehtranslationsbewegungen im Raum zu ermöglichen.

2. Bein-Fuß-Orthese, die am Knöchel angelenkt ist, nach Anspruch 1, wobei der erste Bügel (4) über Schrauben (9) oder geeignete Befestigungsvorrichtungen mit dem Beinhalter (2) verbunden ist, wobei der zweite Bügel (5), auf seiner Oberfläche, den einzelnen bogenförmigen Schlitz (52), das Schlitzloch (53), ein Paar Löcher (56) und einen Schlitz (55) umfasst, wobei die Bein-Fuß-Orthese ferner einen L-Bügel (6) umfasst, der mit der Fersenschale (1) im Eingriff steht und eine Lasche (61) umfasst, an der eine Reihe von Löchern (63) und ein Loch (62) parallel zu diesen vorhanden sind, und wobei das Paar Stifte (7, 8) aus einem Paar zylindrischen Gewindenieten (10, 11), die am ersten Bügel (4) bereitgestellt sind, einem Paar Schrauben (12, 13) und einem Paar Zahnscheiben (14, 15) besteht.

3. Bein-Fuß-Orthese, die am Knöchel angelenkt ist, nach Anspruch 1, ferner umfassend zwischen den Bügeln (4, 5) des Scharniermechanismus (3) eine Distanzscheibe (16), die aus Teflon oder einem anderen Material, das zum Induzieren einer Reibungsreduktion zwischen den Oberflächen der Bügel (4, 5) konzipiert ist, besteht.

4. Bein-Fuß-Orthese, die am Knöchel angelenkt ist, nach Anspruch 2, wobei der Bügel (5) über ein Paar Schrauben (17), die durch das Paar Löcher des Bügels (5) im Eingriff stehen, und eine Schraube (18), die am Schlitz (55) des Bügels (5) festgelegt ist, mit dem L-Bügel (6) und somit mit der Fersenschale (1) verbunden ist, wobei die Schrauben (17, 18) auf geeignete Weise innerhalb der Löcher (62, 63) des L-Bügels (6) verschraubt sind.

5. Bein-Fuß-Orthese, die am Knöchel angelenkt ist, nach einem oder mehreren der vorhergehenden Ansprüche, wobei der erste Bügel (4) und der Beinhalter (2) aus einem Beinhalter (2) bestehen, an dessen Seitenfläche geeignete Elemente vorhanden sind, die über den Scharniermechanismus eine Verbindung des Beinhalters (2) mit der Fersenschale (1) ermöglichen.

6. Bein-Fuß-Orthese, die am Knöchel angelenkt ist, nach einem oder mehreren der vorhergehenden Ansprüche, wobei der zweite Bügel (5), der L-Bügel (6) und die Fersenschale (1) eine Fersenschale (1) umfassen, an deren Seitenfläche geeignete Elemente vorhanden sind, die über den Scharniermechanismus eine Verbindung der Fersenschale (1) mit dem Beinhalter (2) ermöglichen.

7. Bein-Fuß-Orthese, die am Knöchel angelenkt ist, nach einem oder mehreren der vorhergehenden Ansprüche, wobei der erste Bügel (4) ein Bügel (4a) ist, der ein elliptisches Ende (41a) aufweist, das um ungefähr 20° in Bezug auf das andere Ende (42a) geneigt ist, wobei der zweite Bügel (5a) geeignete Befestigungselemente umfasst und ferner eine elliptische Distanzscheibe (16a) umfasst.

## Revendications

1. *Orthèse de jambe-pied, articulée à la cheville, utilisée dans la phase de récupération fonctionnelle après un événement traumatique ou en cas de blessure dans les os du pied ou dans les ligaments croisés de la cheville et de l'arrière-pied,* composée d'une enveloppe de talon (1), d'une jambière (2) et d'un mécanisme d'articulation (3) engagé avec ladite enveloppe de talon (1) et ladite jambière (1), le mécanisme d'articulation (3) comprenant un premier étrier (4) et un second étrier (5), qui sont mécaniquement articulés par le biais d'une première broche (7) et d'une seconde broche (8) respectivement engagées dans les ouvertures (52, 53) fournies dans les étriers (4, 5), **caractérisé en ce que** l'ouverture (52) dans laquelle la première broche (7) est engagée est la première fente (52) façonnée comme des arcs circonférentiels consécutifs, et l'ouverture (53) dans laquelle la seconde broche (8) est engagée est un trou oblong (53), le trou oblong allant de façon transversale vers ladite première fente (52), de telle sorte que ledit mécanisme d'articulation (3) est adapté pour permettre à ladite enveloppe de talon (1) et à ladite jambière (2) d'effectuer des mouvements roto-translatifs simultanés dans l'espace.

2. Orthèse de jambe-pied, articulée à la cheville selon la revendication 1, dans laquelle le premier étrier (4) est raccordé par des vis (9) ou des dispositifs de fixation appropriés à la jambière (2), le second étrier (5) comprenant, sur sa surface, la fente arquée unique (52), le trou oblong (53), une paire de trous (56) et une fente (55), dans lequel l'orthèse jambe-pied comprend en outre un étrier en L (6), engagé avec l'enveloppe de talon (1), et une languette (61) sur laquelle se trouvent une série de trous (63) et un trou (62) qui leur est parallèle, et dans lequel la paire de broches (7, 8) est composée d'une paire de rivets cylindriques filetés (10, 11) fournis sur le premier étrier (4), une paire de vis (12, 13) et une paire de rondelles avec dentelure externe (14, 15).

3. Orthèse de jambe-pied, articulée à la cheville, selon la revendication 1, comprenant en outre, entre les étriers (4, 5) du mécanisme d'articulation (3), un disque séparateur (16) fabriqué en teflon ou d'un autre matériau conçu pour induire une réduction de la friction entre les surfaces des étriers (4, 5).

4. Orthèse de jambe-pied, articulée à la cheville, selon la revendication 2, dans laquelle l'étrier (5) est raccordé à l'étrier en L (6), et donc à l'enveloppe de talon (1), par une paire de vis (17) engagées à travers la paire de trous de l'étrier (5) et une vis (18) raccordée à la fente (55) de l'étrier (5), lesdites vis (17, 18) étant correctement vissées dans les trous (62, 63) de l'étrier en L (6).

5. Orthèse de jambe-pied, articulée à la cheville, selon une ou plusieurs des revendications précédentes, dans laquelle le premier étrier (4) et la jambière (2) sont composés d'une jambière (2) sur la surface latérale de laquelle sont présents des éléments qui permettent de raccorder la jambière (2) à l'enveloppe de talon (1) par le biais du mécanisme d'articulation.

6. Orthèse de jambe-pied, articulée à la cheville, selon une ou plusieurs des revendications précédentes, dans laquelle le premier étrier (5), l'étrier en L (6) et l'enveloppe de talon (1) comprennent une enveloppe de talon (1) sur la surface latérale de laquelle sont présents des éléments appropriés qui permettent de raccorder ladite enveloppe de talon (1) à la jambière (2) par le biais du mécanisme d'articulation.

7. Orthèse de jambe-pied, articulée à la cheville, selon une ou plusieurs des revendications précédentes, dans laquelle le premier étrier (4) est un étrier (4a) ayant une extrémité elliptique (41a), qui est inclinée à environ 20° par rapport à l'autre extrémité (42a), le second étrier (5a) comprenant des éléments de fixation appropriés et comprenant en outre un disque d'entretoise elliptique (16a).
